(19) Europäisches Patentamt
European Patent Office
Office européen des brevets

(11) Veröffentlichungsnummer : **0 325 279 B1**

(12) # EUROPÄISCHE PATENTSCHRIFT

(45) Veröffentlichungstag der Patentschrift :
**01.04.92 Patentblatt 92/14**

(51) Int. Cl.$^5$ : **C07C 401/00**, A61K 31/59, C07F 7/18

(21) Anmeldenummer : **89100974.8**

(22) Anmeldetag : **20.01.89**

(54) **Dehydrocholecalciferolderivate.**

(30) Priorität : **20.01.88 US 145932**

(43) Veröffentlichungstag der Anmeldung :
**26.07.89 Patentblatt 89/30**

(45) Bekanntmachung des Hinweises auf die Patenterteilung :
**01.04.92 Patentblatt 92/14**

(84) Benannte Vertragsstaaten :
**AT BE CH DE ES FR GB GR IT LI LU NL SE**

(56) Entgegenhaltungen :
**EP-A- 0 122 490**
**WO-A-85/03298**
**DE-A- 2 812 741**
**DE-A- 2 920 092**
**DE-A- 3 243 073**
**DE-A- 3 541 934**

(73) Patentinhaber : **F. HOFFMANN-LA ROCHE AG**
**Postfach 3255**
**CH-4002 Basel (CH)**

(72) Erfinder : **Baggiolini, Enrico Giuseppe**
**30 Evergeen Drive**
**North Caldwell, N.J. 07006 (US)**
Erfinder : **Hennessy, Bernard Michael**
**111 Passaic Avenue**
**Nutley, N.J. 07110 (US)**
Erfinder : **Shiuey, Shian-Jan**
**331 Bloomfield Avenue**
**Nutley, N.J. 07110 (US)**
Erfinder : **Truitt, Gary Arthur**
**109 Garner Avenue**
**Bloomfield, N.J. 07003 (US)**
Erfinder : **Uskokovic, Milan Radoje**
**253 Highland Avenue**
**Upper Montclair, N.J. 07043 (US)**

(74) Vertreter : **Mahé, Jean et al**
**Postfach 3255 Grenzacherstrasse 124**
**CH-4002 Basel (CH)**

**EP 0 325 279 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft Verbindungen der Formel

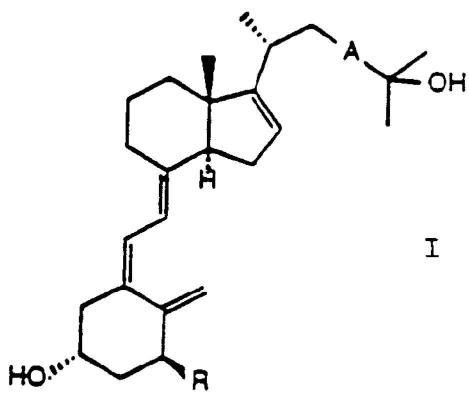

I

worin R Wasserstoff oder Hydroxy und A -C≡C-, -CH=CH- mit E-Konfiguration oder $-CH_2-CH_2-$ ist,

pharmazeutische Präparate enthaltend eine oder mehrere Verbindungen der Formel I und die Verwendung dieser Verbindungen bei der Herstellung von solchen Präparaten für die Behandlung von hyperproliferativen Hautkrankheiten, wie Psoriasis, und für die Behandlung von neoplastischen Krankheiten, wie Leukämie.

Die Erfindung betrifft ferner ein Verfahren zur Herstellung eines Arzneimittels, wobei der Aktivstoff, nämlich eine oder mehrere Verbindungen der Formel I, zusammen mit einem pharmazeutisch geeigneten Trägermaterial und sonstigen Hilfsstoffen in eine für therapeutische Zwecke anwendbare Form gebracht wird, dadurch gekennzeichnet, dass man ein Heilmittel für die Behandlung von hyperproliferativen Hautkrankheiten und von neoplastichen Krankheiten herstellt, bestehend aus der Kombination von einem eine oder mehrere Verbindungen der Formel I enthaltenden Präparat und der Information, welche in direktem Zusammenhang mit der Behandlung der besagten Krankheiten steht.

Beispiele von $C_{1-4}$-Alkylgruppen sind Methyl, Aethyl, Propyl, Isopropyl, Butyl und t-Butyl. Beispiele von Aryl-$C_{1-4}$-alkylgruppen sind Benzyl, Phenethyl und Phenylpropyl. Beispiele von Arylgruppen sind Phenyl und p-Tolyl. Halogen bezeichnet Brom, Chlor, Fluor oder Jod.

Die folgenden Verbindungen A bis F fallen unter die Formel I:

A: 1,25-Dihydroxy-16-dehydrocholecalciferol;
B: 25-Hydroxy-16-dehydrocholecalciferol;
C: 1,25-Dihydroxy-16,23E-bisdehydrocholecalciferol;
D: 25-Hydroxy-16,23E-bisdehydrocholecalciferol;
E: 1,25-Dihydroxy-16-dihydro-23-didehydrocholecalciferol; und
F: 25-Hydroxy-16-dehydro-23-didehydrocholecalciferol;

wobei die 1,25-dihydroxylierten Verbindungen A, C und E bevorzugt sind.

Die Verbindungen der Formel I, d.h. diejenigen der Formeln Ia und Ib, können wie in den Schemen 1, 2 und 3 beschrieben dadurch hergestellt werden, dass man eine entsprechende Verbindung der Formel I, die anstelle von zwei oder drei Hydroxygruppen zwei bzw. drei geschützte Hydroxygruppen der Formel

$$-OSi(R_1,R_2R_3)$$

enthält, worin $R_1$ und $R_3$ $C_{1-4}$-Alkyl und $R_2$ $C_{1-4}$-Alkyl, Aryl oder Aryl-$C_{1-4}$-alkyl darstellt, mit einem für die Abspaltung der Schutzgruppen geeigneten Reagenz umsetzt.

Schema 1

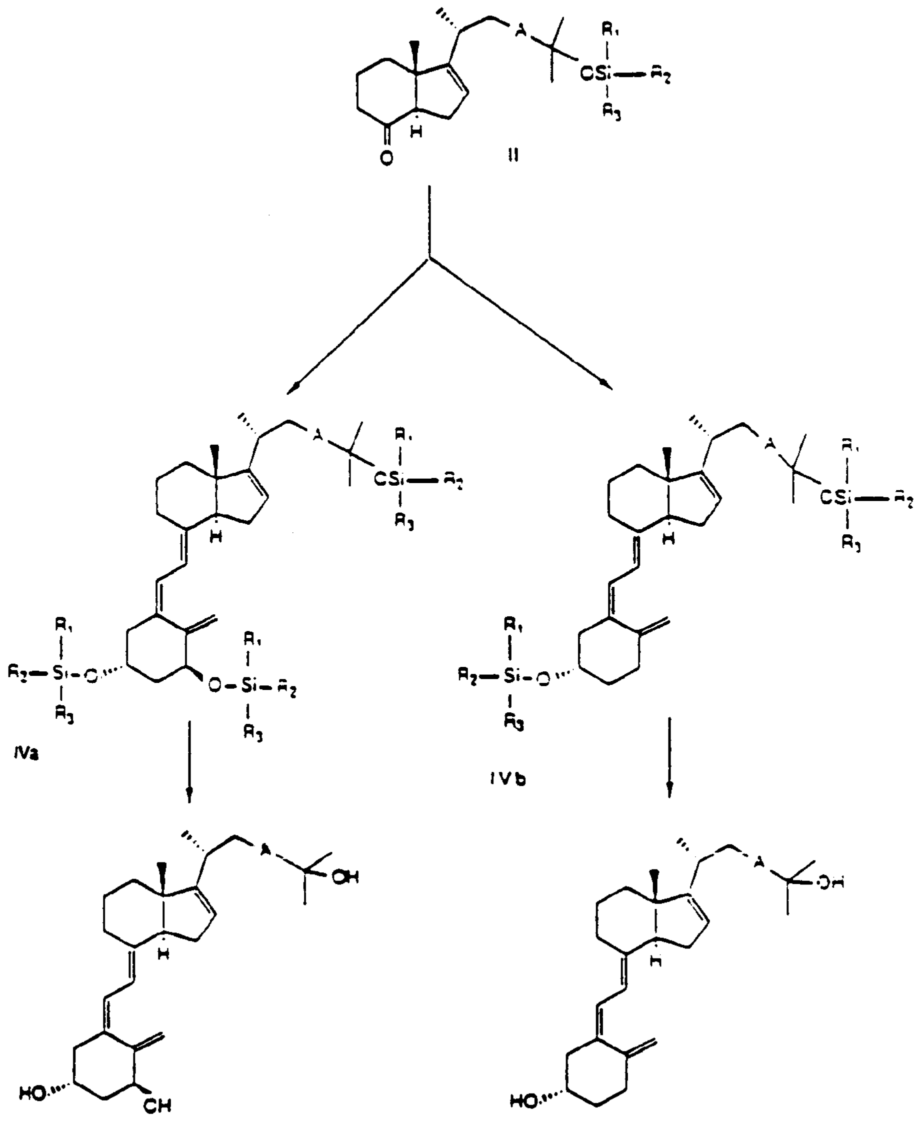

II

IVa

IVb

Ia

Ib

worin A, $R_1$, $R_2$ und $R_3$ die obige Bedeutung haben.

Schema 2

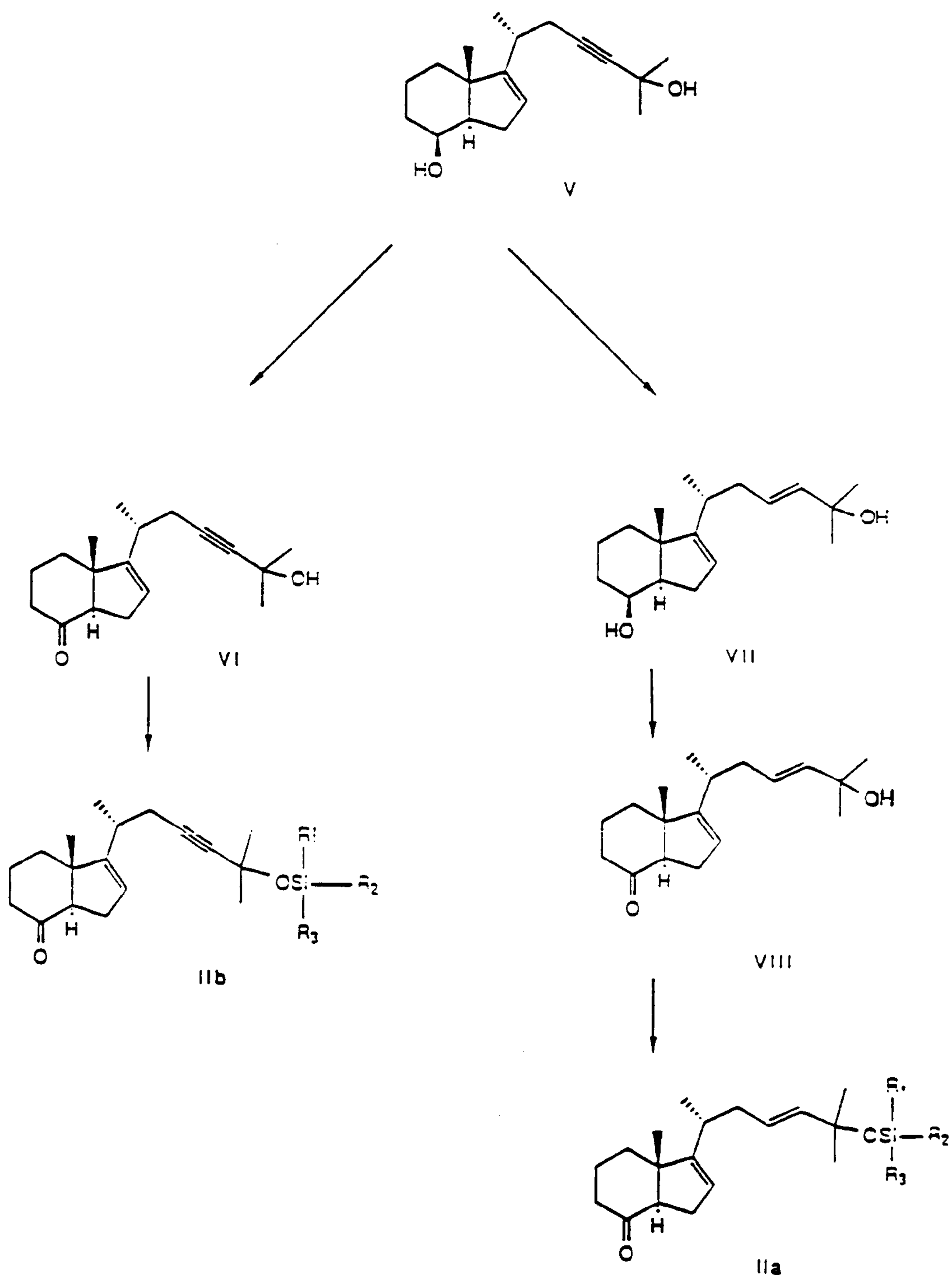

V

VI

VII

IIb

VIII

IIa

worin $R_1$, $R_2$ und $R_3$ die obigen Bedeutungen haben.

## Schema 3

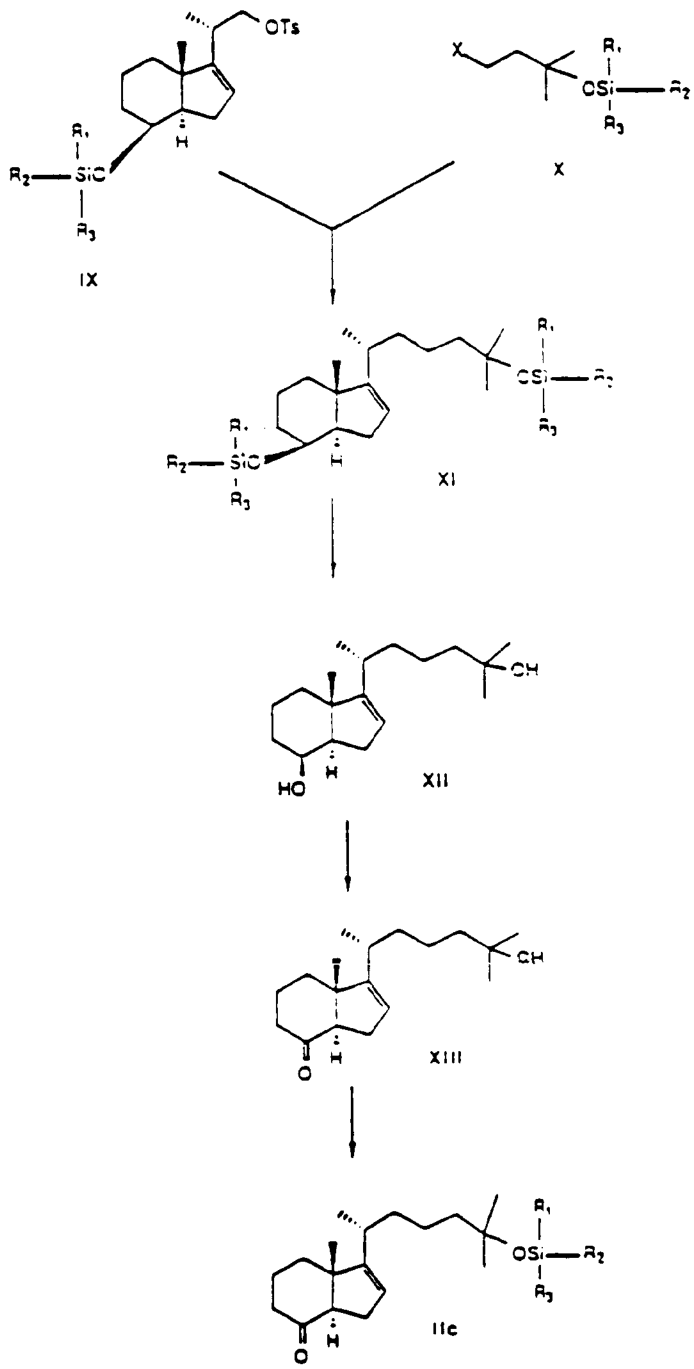

IX X XI XII XIII IIc

worin $R_1$, $R_2$ und $R_3$ die obigen Bedeutungen haben, X Chlor, Brom oder Jod und Ts Tosyl ist.

Die Zwischenprodukte der Formel II, d.h. diejenigen der Formeln IIa, IIb und IIc, sind neu und Gegenstand der Erfindung.

In Schema I wird eine Verbindung der Formel II in eine solche der Formeln IVa oder IVb uebergeführt durch Reaktion mit der entsprechenden Verbindung der Formel

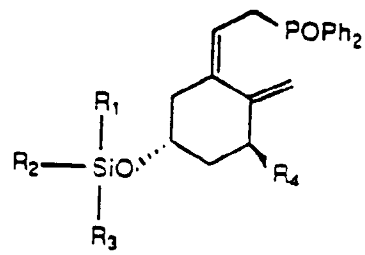

III

worin Ph Phenyl; $R_4$ Wasserstoff oder -OSi($R_1$, $R_2$, $R_3$) ist, und $R_1$, $R_2$ und $R_3$ die obige Bedeutung haben.

Die Reaktion wird bei -60 bis 90°C vorzugsweise -75°C in einem polaren, aprotischen, organischen Lösungsmittel, z.B. trockenem Aether oder vorzugsweise trockenem Tetrahydrofuran (THF) in Gegenwart einer starken Base wie einem, Alkyllithium, z.B. Butyllithium, bewerkstelligt.

Die Schutzgruppen in einer Verbindung der Formel IVa oder IVb werden entfernt durch Reaktion mit einem Fluorsalz, z.B. Tetrabutylammoniumfluorid in einem polaren, organischen Lösungsmittel, z.B. Aether oder vorzugsweise THF. Man erhält die entsprechende Verbindung der Formel Ia oder Ib.

In Schema 2 wird die Verbindung der Formel V zu derjenigen der Formel VI oxidiert. Dazu verwendet man z.B. 2,2′-Bipyridiniumchlorochromat oder vorzugsweise Pyridiniumchlorochromat in einem aprotischen, organischen Lösungsmittel, wie Methylenchlorid.

Die Verbindung der Formel VI wird in eine solche der Formel IIb übergeführt, z.B. durch Reaktion mit einem Trialkylsilylimidazol, wie Trimethylsilylimidazol, in einem aprotischen, organischen Lösungsmittel, wie THF oder vorzugsweise Methylenchlorid.

Die Verbindung der Formel V kann auch zu einer solchen der Formel VII hydriert werden, z.B. durch Reaktion mit einem Reduktionsmittel, wie Lithiumaluminiumhydrid, vorzugsweise in Gegenwart eines Alkalimetallalkoxyds, z.B. Natriummethoxyd, in einem aprotischen, organischen Lösungsmittel, z.B. Aether oder vorzugsweise THF, bei Rückflusstemperatur (etwa 68°C für THF). Die Reaktion dauert etwa 10-20 Stunden.

Die erhaltene Verbindung der Formel VII wird zur Verbindung der Formel VIII oxidiert, wie weiter oben beschrieben für die Oxidation von V zu VI.

Die Verbindung der Formel VIII wird zu einer solchen der Formel IIa übergeführt durch Reaktion mit einem Trialkylsilylimidazol, wie weiter oben beschrieben für die Umwandlung von VI zu IIb.

In Schema 3 eine Verbindung der Formel X in einem Aether, vorzugsweise THF, bei Rückflusstemperatur, mit Magnesium umgesetzt. Die entstandene Grignard-Lösung wird zuerst mit Kupfer(I)-jodid und dann mit der Verbindung der Formel IX versetzt.

Die erhaltene Verbindung der Formel XI wird mit einem Fluorid, z.B. Tetrabutylammoniumfluorid in Aether oder vorzugsweise THF umgesetzt.

Die erhaltene Verbindung der Formel XII kann wie weiter oben beschrieben für die Oxidation von V zu VI oxidiert werden.

Die entstandene Verbindung der Formel XIII wird zu einer solchen der Formel IIc umgesetzt durch Behandlung mit einem Trialkylsilylimidazol wie weiter oben beschrieben für die Umwandlung von VI in IIb.

Zur Herstellung einer Verbindung der Formel IX wird eine Verbindung der Formel

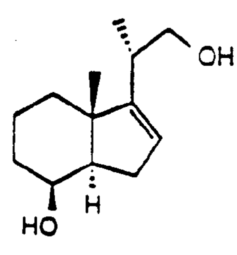

XIV

(Tetrahedron 40, 1984,2283) mit einem Tosylierungsmittel wie einem p-Toluolsulfonylhalogenid, z.B. dem Chlorid, in einer organischen Base, z.B. Collidin oder vorzugsweise Pyridin, umgesetzt. Die entstandene Verbindung der Formel

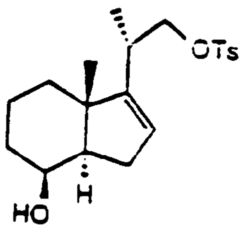

XV

wird dann in eine solche der Formel IX übergeführt durch Reaktion eines Trialkylsilylchlorids, z.B. Trimethylsilylchlorid, in Gegenwart von Imidazol und in einem aprotischen, organischen Lösungsmittels, z.B. THF oder Methylenchlorid.

Zur Herstellung einer Verbindung der Formel X wird eine Verbindung der Formel

$X\text{-}CH_2CH_2COCH_3$ XVI

zu einer solchen der Formel

$X\text{-}CH_2CH_2C(CH_3)_2\text{-}OH$ XVII

worin X die obige Bedeutung hat, übergeführt durch Reaktion mit einem Methyl-Grignard-Reagenz, wie Methylmagnesiumbromid in Aether. Die Verbindung der Formel XVII wird zu einer solchen der Formel X übergeführt durch Reaktion mit einem Trialkylsilylchlorid wie weiter oben beschrieben für die Umwandlung von XV in IX.

Zur Herstellung einer Verbindung der Formel V wird eine Verbindung der Formel XV mit einem Cyanid bildenden Mittel, z.B. Natriumcyanid, in einem aprotischen, organischen Lösungsmittel, z.B. Dimethylsulfoxid (DMSO), bei einer Temperatur zwischen 80 und 100°C während 1-5 Stunden umgesetzt. Man erhält die Verbindung der Formel

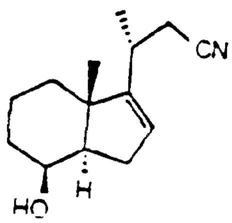

XVIII

Diese wird in die Verbindung der Formel

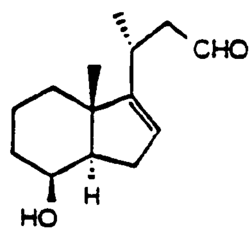

XIX

umgewandelt durch Reaktion mit einem Reduktionsmittel, z.B. Diisobutylaluminiumhydrid, gefolgt durch Hydrolyse, z.B. mit einer anorganischen Säure, wie Salzsäure. Die Reduktion wird einem aprotischen, organischen Lösungsmittel, z.B. Methylenchlorid bei etwa -10 bis 10°C während 20 bis 90 Minuten bewerkstelligt. Die Verbindung der Formel XIX wird in eine solche der Formel

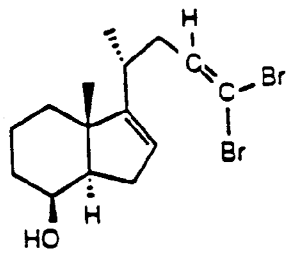

XX

übergeführt durch Reaktion mit einem Gemisch von Triphenylphosphin, Tetrabromkohlenstoff und Zinkstaub in einem aprotischen, organischen Lösungsmittel, z.B. Methylenchlorid, während etwa 1 bis 30 Stunden.

Die Verbindung der Formel XX wird in eine solche der Formel

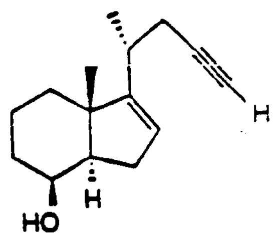

XXI

überfeführt durch Reaktion mit einer starken Base, z.B. Butyllithium in einem polaren aprotischen Lösungsmittel, z.B. THF, bei etwa -80 bis -70°C während etwa 1 bis 3 Stunden. Die Verbindung der Formel XXI wird in diejenige der Formel

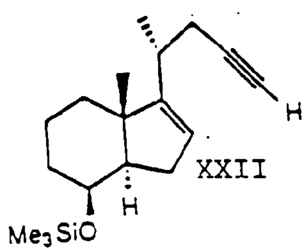

XXII

übergeführt durch Reaktion mit Trimethylsilylimidazol in einem aprotischen, organischen Lösungsmittel, z.B. THF oder Methylenchlorid. Diese Verbindung wird in diejenige der Formel

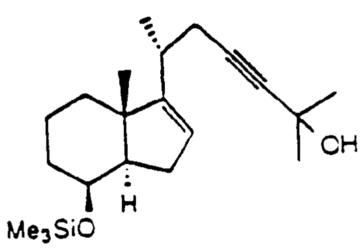

XXIII

übergeführt durch Reaktion mit einer starken Base, z.B. Butyllithium, und dann mit Aceton. Die Reaktion wird in einem aprotischen, organischen Lösungsmittel, z.B. THF, bei etwa -80° bis -60°C durchgeführt. Die Verbindung der Formel XXIII wird in diejenige der Formel V (im Schema 2) übergeführt durch Reaktion mit einem Fluorid, z.B. Tetrabutylammoniumfluorid, in einem organischen Lösungsmittel, z.B. Aether oder THF.

Die Verbindungen der Formel I stimulieren die Differenzierung und verringern die Proliferation der menschlichen Keratinocyten. Daher können sie verwendet werden als Mittel zur Behandlung von hyperproliferativen Hautkrankheiten, wie Psoriasis, Basalzellencarcinomen, Keratinisierungsstörungen und Keratose. Die Verbindungen der Formel I sind auch verwendbar als Mittel zur Behandlung von neoplastischen Krankheiten, wie Leukämie.

Die Aktivität der Verbindungen der Formel I als Mittel zur Behandlung von hyperproliferativen Hautkrankheiten kann nach an sich bekannten Methoden, z.B. wie in The Society for Investigative Dermatology 1986, 709-714 beschrieben, nachgewiesen werden. Die Effekte der Verbindungen A bis F auf die morphologische Differenzierung von gezüchteten menschlichen Keratinocyten, verglichen mit dem Effekt von 1,25-Dihydroxycholecalciferol (Verbindung X) ist in den Tabellen 1 bis 4 als Anzahl ($x10^4$) gezüchteter menschlicher Keratinocyten angegeben. Eine Verbindung, die die Differenzierung von Basalzellen zu Plattenepithelzellen und verhornte Plattenepithelzellen induziert, ist geeignet für die Behandlung von Hautkrankheiten, die gekennzeichnet sind durch Keratinisierungsstörungen, wie Psoriasis.

## Tabelle 1

| Verbindung | Dosis (M) | Anzahl Zellen ($x10^4$): Gesamtanzahl | Basalzellen | Plattenepithelzellen | verhornte Plattenepithelzellen |
|---|---|---|---|---|---|
| Kontrolle | | 133±5 | 118±4 | 15±1 | 18±2 |
| X | $10^{-10}$ | 122±4 | 103±2 | 19±2 | 23±1 |
| | $10^{-8}$ | 112±6 | 89±2 | 23±4 | 30±3 |
| | $10^{-6}$ | 95±7 | 64±6 | 31±1 | 34±2 |
| A | $10^{-10}$ | 132±8 | 115±7 | 17±1 | 27±2 |
| | $10^{-8}$ | 128±10 | 106±8 | 22±2 | 33±2 |
| | $10^{-6}$ | 101±7 | 71±5 | 30±2 | 39±2 |
| B | $10^{-10}$ | 133±6 | 115±5 | 18±1 | 25±1 |
| | $10^{-8}$ | 131±4 | 109±2 | 22±2 | 29±2 |
| | $10^{-6}$ | 104±4 | 74±3 | 30±1 | 33±1 |

Tabelle 2

| Verbindung | Dosis (M) | Anzahl Zellen ($x10^4$): Gesamtanzahl | Basalzellen | Plattenepithelzellen | verhornte Plattenepithelzellen |
|---|---|---|---|---|---|
| Kontrolle | | 123±7 | 105±6 | 18±1 | 74±7 |
| X | $10^{-10}$ | 116±9 | 95±8 | 21±1 | 91±4 |
| | $10^{-8}$ | 101±10 | 75±8 | 26±2 | 122±11 |
| | $10^{-6}$ | 83±5 | 57±4 | 26±1 | 146±16 |
| C | $10^{-10}$ | 117±4 | 92±2 | 25±2 | 103±6 |
| | $10^{-8}$ | 108±3 | 80±2 | 28±1 | 128±3 |
| | $10^{-6}$ | 80±7 | 54±6 | 26±1 | 153±1 |
| D | $10^{-10}$ | 113±7 | 93±6 | 20±1 | 104±10 |
| | $10^{-8}$ | 111±7 | 86±3 | 25±2 | 128±5 |
| | $10^{-6}$ | 94±3 | 68±1 | 26±2 | 144±7 |

Tabelle 3

| Verbindung | Dosis (M) | Anzahl Zellen ($x10^4$): Gesamtanzahl | Basalzellen | Plattenepithelzellen | verhornte Plattenepithelzellen |
|---|---|---|---|---|---|
| Kontrolle | | 108±10 | 93±8 | 15±2 | 88±8 |
| X | $10^{-10}$ | 106±7 | 86±6 | 18±1 | 100±9 |
| | $10^{-8}$ | 84±8 | 61±5 | 23±3 | 122±8 |
| | $10^{-6}$ | 73±7 | 51±5 | 22±2 | 142±11 |
| E | $10^{-10}$ | 86±4 | 63±2 | 23±2 | 114±5 |
| | $10^{-8}$ | 82±3 | 53±2 | 29±1 | 141±5 |
| | $10^{-6}$ | 78±3 | 41±1 | 27±2 | 147±4 |
| F | $10^{-10}$ | 103±5 | 81±3 | 22±2 | 103±4 |
| | $10^{-8}$ | 97±3 | 67±2 | 29±1 | 121±6 |
| | $10^{-6}$ | 84±4 | 55±2 | 29±1 | 137±7 |

Die Aktivität der Verbindungen der Formel I als Mittel für die Behandlung von hyperproliferativen Hautkrankheiten kann auch durch Ermittlung der Anzahl von menschlichen Keratinocyten, von verhornten Plattenepithelzellen und von Plattenepithel-Karzinomzellenlinien (SCC-115), die sich in Gegenwart der besagten Verbindungen bilden.

Die Resultate sind in den Tabellen 4 und 5 angegeben:

Tabelle 4

| Behandlung | Dosis (M) | Anzahl Keratinocten ($x10^4$) | Anzahl verhornte Plattenepithelzellen ($x10^2$) |
|---|---|---|---|
| Kontrolle (0,1% Aethanol) | | 189.49±22.3 | 858.28±185.70 |
| Verbindung E | $10^{-12}$ | 187.36±15.33 | 1136.63± 383.66 |
| | $10^{-10}$ | 175.34±10.19 | 1444.87± 312.47 |
| | $10^{-8}$ | 145.79±15.66 | 2113.62±1049.33 |
| | $10^{-6}$ | 41.95± 7.53 | 1916.83± 887.66 |
| Kontrolle (0,1% Aethanol) | | 148.73±16.23 | 2193.7± 921.9 |
| Verbindung A | $10^{-12}$ | 114.91±10.95 | 1662.2± 420.1 |
| | $10^{-10}$ | 130.37±24.32 | 3973.8± 126.99 |
| | $10^{-8}$ | 120.67±16.87 | 7235.2± 55.5 |
| | $10^{-6}$ | 109.22±15.87 | 8323.5± 157.6 |

Tabelle 5

| Behandlung | Dosis (M) | Anzahl Zellenlinien (SCC-15) ($x10^5$) |
|---|---|---|
| Kontrolle | | 7.35±1.75 |
| Verbindung E | $10^{-12}$ | 6.98±1.68 |
| | $10^{-10}$ | 5.89±1.58 |
| | $10^{-8}$ | 5.76±1.53 |
| | $10^{-6}$ | 0.40±0.98 |
| Verbindung A | $10^{-6}$ | 0.49±0.13 |

Die obigen Resultate zeigen, dass die Verbindungen der Formel I die Differenzierung von Hautzellen induzieren und daher in der Behandlung von hyperproliferativen Hautkrankheiten, wie Psoriasis, verwendbar sind.

Zum Nachweis der Aktivität der Verbindungen der Formel I als Mittel zur Behandlung von neoplastischen Krankheiten wurden die antiproliferativen (AP) und die Differenzierung induzierenden Effekte (DI) der Verbindungen A bis F sowie die Anzahl menschlicher promyelocytischer Tumorzellen HL-60 ermittelt. In Tabelle 6 ist der AP Effekt als prozentuale Reduktion der Anzahl Zellen und als Konzentration $ID_{50}$, die die Zellenanzahl um 50% reduziert, angegeben. Der DI Effekt ist angegeben als Prozentsatz der differenzierten Zellen und als Konzentration $ED_{50}$ der Verbindungen, die eine 50%ige Differenzierung der Zellen bewirkt.

Tabelle 6

| Konzentration $ED_{50}$ (x10 M) | % Reduktion der Anzahl Zellen | $ID_{50}$ (x $10^{-8}$M) | Differenzierte Zellen (%) | $ED_{50}$ (x$10^{-8}$M) |
|---|---|---|---|---|
| Verbindung X | | | | |
| 0.01 | 6 | | 3 | |
| 0.1 | 5 | | 11 | |
| 1 | 16 | 2 | 19 | 2 |
| 10 | 66 | | 68 | |
| 100 | 84 | | 98 | |
| Verbindung A | | | | |
| 0.01 | 10 | | 3 | |
| 0.1 | 33 | | 16 | |
| 1 | 84 | 0.2 | 92 | 0.2 |
| 10 | 85 | | 97 | |
| 100 | 85 | | 98 | |
| Verbindung B | | | | |
| 0.1 | 10 | | 5 | |
| 1 | 8 | | 4 | |
| 10 | 14 | 35 | 6 | 32 |
| 100 | 82 | | 93 | |
| 1000 | 95 | | 95 | |

| | | | | |
|---|---|---|---|---|
| Verbindung C | | | | |
| 0.01 | 18 | | 3 | |
| 0.1 | 20 | | 19 | |
| 1 | 81 | 0.3 | 92 | 0.3 |
| 10 | 85 | | 97 | |
| 100 | 86 | | 99 | |
| Verbindung D | | | | |
| 0.1 | 12 | | 1 | |
| 1 | 12 | | 2 | |
| 10 | 17 | 150 | 17 | 200 |
| 100 | 46 | | 31 | |
| 1000 | 95 | | 97 | |
| Verbindung E | | | | |
| 0.01 | 6 | | 9 | |
| 0.1 | 59 | | 50 | |
| 1 | 80 | 0.07 | 96 | 0.1 |
| 10 | 81 | | 98 | |
| Verbindung F | | | | |
| 0.1 | 13 | | 4 | |
| 1 | 10 | | 12 | |
| 10 | 8 | 70 | 21 | 70 |
| 100 | 58 | | 55 | |
| 1000 | 95 | | 91 | |

Diese Resultate zeigen, dass die Verbindungen der Formel I die Proliferation der menschlichen promyelocytischen Zellen in vitro hemmen, und dabei keine Zelltoxizität aufweisen. Ferner differenzieren die Zellen zu einem reiferen Phänotyp bei den gleichen Dosen, die die Proliferation hemmen. Aus diesen Resultaten ergibt sich, dass diese Verbindungen verwendbar sind als Mittel für die Behandlung von neoplastischen Krankheiten, wie Leukämie.

Die Verbindungen der Formel I können oral verabreicht werden für die Behandlung von neoplastischen Krankheiten oder für die Behandlung von hyperproliferativen Hautkrankheiten bei Warmblütern, die eine solche Behandlung benötigen, z.B. beim Erwachsenen in einer Dosierung im Bereich von etwa 0,1 - 10 mg pro Tag.

Für die Behandlung von hyperproliferativen Hautkrankheiten kann man die Verbindungen der Formel I auch topisch an Warmblüter verabreichen in einer täglichen Dosis von etwa 1 bis 1000 μg/g topische Verabreichungsform.

Zur oralen Verabreichung können die Verbindungen der Formel I z.B. in Kapseln oder Tabletten zusammen

mit pharmazeutisch verwendbaren Trägern verarbeitet werden. Beispiele von solchen Träger für Kapseln sind Bindemittel, wie Tragackanth, Gummi oder Gelatine; Excipienten, wie Dikalziumphosphat; Sprengmittel, wie Maisstärke; Gleitmittel, wie Magnesiumstearat; Süsstoffe, wie Sucrose; Aromastoffe, wie Pfefferminz. Tabletten können mit Shellack, Zucker oder beidem überzogen sein. Ein Sirup oder Elixir kann ein Süssstoff, Methyl- und Propylparabene als Konservierungsmittel, ein Farbstoff und einen Aromastoff enthalten.

Topische Dosierungsformen enthalten Verbindungen der Formel I sind Salben und Creme bestehend aus öligen, resorbierbaren, wasserlöslichen und emulsionsartigen Basen, wie Lanolin und Polyäthylenglycol. Weitere topische Dosierungsformen sind Gele, Lotionen, Pulver und Aerosole. Die topischen Präparate können auch in der Behandlung von Haut- und Schleimhautentzündungen, z.B. der Mundhöhle oder des Dickdarms verwendet werden.

Lotionen, d.h. flüssige Präparate, wie Lösungen bzw. wässrige oder hydroalkoholische Präparate, die die Substanz in Pulverform enthalten, können Suspendierungs- oder Dispergiermittel, wie Zellulosederivate, z.B. Aethyl- oder Methylzellulose; Gelatine oder Gummi, sowie ein Vehikel, wie Wasser, Alkohol oder Glycerol, neben dem Wirkstoff enthalten. Gele sind halbfeste Präparate, die aus einer Lösung oder einer Suspension des Wirkstoffs in einem Vehikel hergestellt werden. Die wässrigen oder wasserfreien Vehikel werden mit einem Gelierungsmittel behandelt, z.B. Carboxypolymethylen, und mit einer Base neutralisiert, z.B. Natriumhydroxid oder einem Amin wie Polyäthylencocoamin.

Beispiel 1

a) Ein Gemisch von 3,24 g [1(R*),3aR*-(3aβ,4α,7aα)]3a,4,-5,6,7,7a-hexahydro-4-hydroxy-β,7a-dimethyl-3H-inden-1-äthanol, 30 ml Pyridin und 3,51 g p-Toluolsulfonylchlorid wird 18 Stunden bei 0°C gerührt. Nach Zusatz von eis und Verdünndung mit Wasser wird das Gemisch mit Methylenchlorid extrahiert. Die organische Phase wird mit 1N $H_2SO_4$ und gesättigtem $NaHCO_3$ gewaschen, dann getrocknet und eingedampft. Der Rückstand wird über Silicagel mit Aethylacetat-Hexan (1:1,5) chromatographiert. Man erhält 4,61 g (82%) [1(R*),3aR*-(3aβ,4α,7aα)]-3a,4,5,6,7a-hexahydro-4-hydroxy-ß,7a-dimethyl-3H-inden-1-äthanol-4-methylbenzolsulfonat, $[\alpha]^{21}_D$ +31.9° (c 0,53, $CHCl_3$).

b) Einer Lösung von 4,61 g des Produkts von a) in 22 ml DMSO werden 1,10 g Natriumcyanid zugesetzt und das Gemisch wird 2 Stunden bei 90°C erhitzt. Nach Abkühlen auf Raumtemperatur wird das Lösungsmittel unter vermindertem Druck entfernt. Das Gemisch wird mit Wasser verdünnt und dann mit Aether extrahiert. Die organische Phase wird mit gesättigter Salzlösung gewaschen, getrocknet und eingedampft. Der Rückstand wird an Silikagel mit Methylenchlorid-Hexan-Aethylacetat (86:7:7) chromatographiert. Man erhält 2,52 g (91%) [1(R*),3aR*-(3aβ,4α,7aα)]-3a,4,5,6,7,7a-hexahydro-4-hydroxy-β,7a-dimethyl-3H-inden-1-propannitril, $[\alpha]^{21}_D$ +29.2° (c 0,65, $CHCl_3$).

c) Einem Gemisch von 6,85 ml Diisobutylaluminiumhydrid in Hexan und 5,2 ml Methylenchlorid wird bei -6°C eine Lösung von 0,430 g des Produkts von b) in 10 ml Methylenchlorid zugesetzt. Das Gemisch wird 55 Minuten bei -6°C gerührt. Nach Zusatz von gesättigtem Ammoniumchlorid wird das Gemisch mittels 3N HCl-Aether (2:1) hydrolisiert. Die wässrige Schicht wird mit Aether extrahiert. Die organischen Schichten werden mit gesättigter Salzlösung gewaschen, getrocknet und eingedampft. Der Rückstand wird an Silikagel mit Aethylacetat-Hexan (1:2) chromatographiert. Man erhält 260 mg (60%) [1(R*),3aR*-(3aβ,4α,7aα)]-3a,4,5,6,7,7a-hexahydro-4-hydroxy-β,7a-dimethyl-3H-inden-1-propanal, $[\alpha]^{22}_D$ +43,1° (c 0,32, $CHCl_3$).

d) Ein Gemisch von 1,77 g Triphenylphosphin, 2,23 g Tetrabromkohlenstoff, 441 mg Zinkstaub und 23 ml Methylenchlorid wird 31 Stunden bei 25°C gerührt. Dem Gemisch wird eine Lösung von 0,430 g des Produkts von c) in 38 ml Methylenchlorid zugesetzt und das Gemisch wird 18 Stunden gerührt. Das Gemisch wird mit Pentan verdünnt und unlösliches Material wird abfiltriert. Die unlösliche Fraktion wird in Methylenchlorid gelöst und die Lösung wird nochmals mit Pentan verdünnt. Nach Filtration werden die vereinigten Filtrate eingedampft. Der Rückstand wird an Silikagel mit 1:4 Aethylacetat-Hexan gereinigt. Man erhält 0,490 g (67%) [1(R*),3aR*-(3aβ,4α,7aα)]-1-(4,4-Dibrom-1-methyl-3-butenyl)-3a,4,5,6,7,7a-hexahydro-7a-methyl-3H-inden-4-ol, $[\alpha]^{22}_D$ +14,4° (c 0,55, $CHCl_3$).

e) Einer Lösung von 0,680 g des Produkts von d) in 31 ml THF werden bei -75°C 3,77 ml einer 1,6M Lösung von Butyllithium in Hexan zugetropft. Das Gemisch wird 1 Stunde bei -75° und 1 Stunde bei 25°C gerührt. Nach Zusatz von gesättigter Salzlösung wird das Gemisch mit gesättigtem wässrigem $NaHCO_3$ verdünnt und mit Aether extrahiert. Die organische Phase wird mit gesättigter Salzlösung gewaschen, getrocknet und eingedampft. Der Rückstand wird über Silikagel mit Aethylacetat-Hexan (1:4) chromatographiert. Man erhält 0,350 g (89%) [1(R*),3aR*-(1-Methyl-3-butynyl)-3H-inden-4-ol, $[\alpha]^{22}_D$ +30,7° (c 0,42, $CHCl_3$).

f) Einer Lösung von 1,29 g des Produkts von e) in 80 ml Methylenchlorid werden 3,59 g 1-(Trimethylsilyl)imidazol zugesetzt. Das Gemisch wird 3 Stunden gerührt. Nach Zusatz von 40 ml Wasser und Rühren während 20 Minuten wird das Gemisch mit Aethylacetat extrahiert. Die organische Phase wird mit Wasser und gesättigter Salzlösung gewaschen, getrocknet und eingedampft. Der Rückstand wird an Silikagel mit Aethylacetat-Hexan (1:15) gereinigt. Man erhält 1,70 g (99%) [1(R*),3aR*-(3aβ,4α,7aα)]-3a,4,5,6,7,7a-hexahydro-7a-methyl-1-(1-methyl-3-butynyl)-4-[(trimethylsilyl)oxy]-3H-inden, $[\alpha]^{20}_{D}$ +39,7° (c 0,30, $CHCl_3$).

g) Einer Lösung von 1,70 g des Produkts von f) in 48 ml THF werden bei -75°C 6,01 ml 1,6M Butyllithium in Hexan zugetropft. Nach 40 Minuten Rühren werden 3.05 ml Aceton zugesetzt und das Gemisch wird 20 Minuten bei -75°C und 75 Minuten bei 25°C gerührt. Nach Zusatz von 40 ml eines 1:1-Gemisches von 2M $KHCO_3$ und 1M Kaliumnatriumtartrat wird das Gemisch 20 Minuten gerührt und dann mit Aethylacetat extrahiert. Die organische Phase wird mit gesättigter Salzlösung gewaschen, getrocknet und eingedampft. Der Rückstand wird an Silikagel mit Aethylacetat-Hexan (1:5) chromatographiert. Man erhält 1,62 g (89%) [1(R*),3aR*-(3aβ,4α,7aα)]-6-(3a,4, 5,6,7,7a-hexahydro-7a-methyl-4-[(trimethylsilyl)-oxy]-3H-inden-1-yl)-2-methyl-3-heptyn-1-ol, $[\alpha]^{20}_{D}$ +39,7° (c 0,30, $CHCl_3$).

h) Einer Lösung von 1,62 g des Produkts von g) in 53 ml THF werden 15,5 ml 1M Tetrabutylammoniumfluorid in THF zugesetzt. Das Gemisch wird 50 Minuten gerührt. Nach Verdünnen mit halbkonzentriertem $NaHCO_3$ wird das Gemisch eingedampft zur Entfernung des grössten Teils des Lösungsmittels und mit Aethylactetat extrahiert. Die organische Phase wird mit halb konzentrierter Salzlösung gewaschen, getrocknet und eingedampft. Der Rückstand wird an Silikagel mit Aethylacetat-Hexan (1:1) chromatographiert. Man erhält 1,17 g (82%) [1(R*),3aR*-(3aβ,4α,7aα)]-3a,4,5,6,7,7a-Hexahydro-1-(1,5-dimethyl-5-hydroxy-3-hexynyl)-7a-methyl-3H-inden-4-ol, Schmelzpunkt 105-107°C.

i) Einer Lösung von 0,720 g des Produkts von h) in 44 ml Methylenchlorid werden 1,59 g Natriumacetat und 3,18 g 2,2′-Bipyridiniumchlorochromat zugesetzt. Das Gemisch wird 2 Stunden gerührt. Nach Zusatz von 1,59 g 2,2′-Bipyridiniumchlorochromat wird das Rühren 2 Stunden fortgesetzt. Nach Zusatz von 6 ml 2-Propanol wird das Gemisch mit Wasser verdünnt und mit Aether-Aethylacetat (1:1) extrahiert. Die organische Phase wird mit Wasser, 1N $H_2SO_4$, gesättigter $NaHCO_3$ und gesättigter Salzlösung gewaschen. Die Lösung wird eingedampft und der Rückstand über Silikagel mit Aethylacetat-Hexan (1:1) chromatographiert. Man erhält 0,560g (78%) [1(R*),3aR*-(3aβ,7aα)]-3,3a,5,6,7,7a-Hexahydro-1-(5-hydroxy-1,5-dimethyl-3-hexynyl)-7a-methyl-4H-inden-4-on, $[\alpha]^{20}_{D}$ +35,3° (c 0,36, $CHCl_3$).

j) Einer Lösung von 0,552 g des Produkts von i) in 70 ml Methylenchlorid werden 2,00 g 1-(Trimethylsilyl)imidazol zugesetzt. Nach 17 Stunden Rühren und Zusatz von 22 ml Wasser wird das Gemisch mit Aethylacetat extrahiert. Die organische Phase wird mit Wasser und gesättigter Salzlösung gewaschen, dann getrocknet und eingedampft. Der Rückstand wird an Silikagel mit Aethylacetat-Hexan (1:4) chromatographiert. Man erhält 0,693 g (99%) [1(R*),3aR*-(3aβ,7aα)]-3,3a,5,6,7,7a-Hexahydro-1-(1,5-dimethyl-5-[(trimethylsilyl)oxy]-3-hexynyl)-7a-methyl-4H-inden-4-on, $[\alpha]^{20}_{D}$ +29,5 ° (c 0,20 $CHCl_3$).

k) Einer Lösung von 2,00 g [3S-(1Z,3α,5β)]-[2-[3,5-Bis-[[(1,1-dimethyläthyl)dimethylsilyl]oxy]-2-methylencyclohexyliden]äthyl] diphenylphosphinoxid in 45 ml THF werden bei -75°C 1,87 ml 1,6M Butyllithium in Hexan zugetropft. Nach 6 Minuten Rühren wird eine Lösung 0,693 g des Produkts von j) in 26 ml THF zugetropft. Nach 70 Minuten Rühren bei -75°C und Zusatz von einem 1:1-Gemisch von 1M Kaliumnatriumtartrat und 2M $KHCO_3$ wird das Gemisch mit Aethylacetat extrahiert. Die organische Phase wird mit gesättigter Salzlösung gewaschen, getrocknet und eingedampft. Der Rückstand wird an Silikagel mit Aethylacetat-Hexan (1:15) chromatographiert. Man erhält 1,23 g (87%) (1α,3β,5Z,7E)-1,3-Bis[[1,1-dimethyläthyl)dimethylsilyl]-oxy]-25-[(trimethylsilyl)oxy]-9,10-secocholesta-5,7,10(19),16-tetraen-3-yn, $[\alpha]^{23}_{D}$ +47,1° (c 0,21 $CHCl_3$).

l) Einer Lösung von 0,228 g des Produkts von k) in 11 ml THF werden 1,92 ml 1M Tetrabutylammoniumfluorid in THF zugesetzt. Das Gemisch wird 16 Stunden gerührt. Nach Verdünnung mit Wasser wird das Gemisch mit Aethylacetat extrahiert. Die organische Phase wird mit halb gesättigter Salzlösung und gesättigter Salzlösung gewaschen, getrocknet und eingedampft. Der Rückstand wird an Silikagel mit Aethylacetat-Hexan (3:1) gereinigt. Man erhält 0,126 g (96%) 1,25-Dihydroxy-16-dehydro-23-didehydrocholecalciferol, $[\alpha]^{21}_{D}$ +21,5 (c 0,20, MeOH).

Beispiel 2

a) Wie in Beispiel 1k) beschrieben, jedoch ausgehend von 0,343 g [5S-(1Z)]-[2-[5-[[(1,1-Dimethyläthyl)dimethylsilyl]oxy]-2-methylencyclohexyliden]äthyl]diphenylphosphinoxid und 0,186 g des Produkts von Beispiel 1j) erhält man 0,205 g (80%) (3β,5Z,7E)-3-[[(1,1-Dimethyläthyl)dimethylsilyl]oxy]-25-[(trimethylsilyl)oxy]-9,10-secocholesta-5,7,-10(19),16-tetraen-23-yn, MS m/e 580 ($M^+$).

b) Durch behandlung von 0,248 g des Produkts von a) wie beschrieben in Beispiel 11) erhält man 0,153 g (91%) 25-Hydroxy-16-dehydro-23-didehydrocholecalciferol, $[\alpha]_D^{21}$ +99,6° (c 0,25), MeOH).

Beispiel 3

a) Einem Gemisch von 0,146 g Lithiumaluminiumhydrid, 0,211 g Natriummethoxid und 6,5 ml THF werden bei 0°C eine Lösung von 0,180 g des Produkts von Beispiel 1h) in 13 ml THF zugetropft. Das Gemisch wird 16 Stunden bei 68°C erhitzt und dann auf 0°C abgekühlt. Nach Verdünnung mit 13 ml Aether und Zusatz von 0,30 ml Wasser und 0,26 ml 10%igem wässrigen NaOH wird das Gemisch 1 Stunde bei Raumtemperatur gerührt und filtriert. Die Feststoffe werden mit Aether vermischt und filtriert. Die vereinigten Filtrate werden eingedampft und an Silikagel mit Aethylacetat-Hexan (1:2) chromatographiert und man erhält 0,179 g (99%) [1(R*),1-(3E),3aβ,4α,7aα)]-(3a,4,5,6,7,7a-Hexahydro-1-(5-hydroxy-1,5-dimethyl-3-hexenyl)-7a-methyl-1H-inden-4-ol, $[\alpha]_D^{21}$ +11,5° (c 0,33, $CHCl_3$).

b) Einer Lösung von 0,120 g des Produkts von a) in 10 ml Methylenchlorid werden 0,500 g Pyridiniumdichromat und 25 mg Pyridinium-p-toluolsulfonat zugesetzt. Das Gemisch wird 135 Minuten gerührt. Nach Zusatz von 40 ml Aether wird das Gemisch 5 Minuten gerührt und filtriert. Die Feststoffe werden mit Aether gemischt und filtriert. Die vereinigten Filtrate werden mit gesättigtem wässrigem $CuSO_4$, Wasser, halbgesättigtem wässrigem $NaHCO_3$ und gesättigter Salzlösung gewaschen. Die organische Phase wird getrocknet und eingedampft. Der Rückstand wird an Silikagel mit 35% Aethylacetat-Hexan chromatographiert. Man erhält 90 mg (76%) [1(R*),1(3E),(3aβ,7aα]-3,3a,5,6,7,7a-Hexahydro-1-(5-hydroxy-1,5-dimethyl-3-hexenyl)-7a-methyl-4H-inden-4-on, $[\alpha]_D^{25}$ +30,6° (c 0,17, $CHCl_3$).

c) Durch Behandlung von 0,099 g des Produkts von b) wie in Beispiel 1j) beschrieben erhält man 0,111 g (89%) [1(R*),-1(3E),(3aβ,7aα)]-3,3a,5,6,7,7a-Hexahydro-1-(1,5-dimethyl-5-[(trimethylsilyl)oxy]-3-hexenyl)-7a-methyl-4H-inden-4-on, $[\alpha]_D^{24}$ +26,4° (c 0,22, $CHCl_3$).

d) Wie in Beispiel 1k) beschrieben erhält man ausgehend von 0,265 g [3S-(1Z,3α,5β)]-[2-[3,5-Bis[[(1,1-dimethyläthyl)dimethylsilyl]oxy]-2-methylencyclohexyliden]äthyl]diphenylphosphinoxid und 0,095 g des Produkts von c), 0,162 g (83%) (1β,3α,5Z,7E,23E)-1,3-Bis[[(1,1-dimethyläthyl)dimethylsilyl]oxy]-25-[(trimethylsilyl]oxy]-9,10-secocholesta-5,7,10(19),16,23-pentaen, MS m/e 712 ($M^+$).

e) Durch Behandlung von 0,159 g des Produkts von d) wie beschrieben in Beispiel 11) erhält man 0,077 g (84%) 1,25-Dihydroxy-16,23E-bisdehydrochlolecaldiferol, $[\alpha]_D^{24}$ + 46,5° (c 0,20, MeOH).

Beispiel 4

a) Wie beschrieben in Beispiel 1k) erhält man ausgehend von 0,225 g [5S-(1Z)]-[2-[5-[[(1,1-Dimethyläthyl)dimethylsilyl]oxy]-2-methylencyclohexyliden]äthyl]diphenylphosphinoxid und 0,110 g des Produkts von Beispiel 3c), 0,150 g (81%) (3β,5Z,7E,23E)-3-[[(1,1-Dimethyläthyl)dimethylsilyl]oxy]-25-[(trimethylsilyl]oxy]-9,10-secocholesta-5,7,-10(19),16,23-pentaen, $[\alpha]_D^{24}$ + 68.3° (c 0,18, $CHCl_3$).

b) Durch Behandlung von 0,144 g des Produkts von a) wie beschrieben in Beispiel 11) erhält man 0,076 g (78%) 25-Hydroxy-16,23E-bisdehydrocholecalciferol, $[\alpha]_D^{22}$ + 62,5° (c 0,20, MeOH).

Beispiel 5

a) Einer Lösung von 6,25 g 3-Bromäthylpropionat in 28 ml THF werden bei -20°C 28,8 ml 2,8 M Methylmagnesiumbromid in Aether zugesetzt. Das Gemisch wird 170 Minuten bei Raumtemperatur gerührt. Nach Zusatz von 15 ml gesättigtes wässriges Ammoniumchlorid und 42 ml 1N HCl wird die organische Phase abgetrennt und die wässrige Phase mit Aether extrahiert. Die organischen Extrakte werden mit gesättigter Salzlösung gewaschen, getrocknet und eingedampft. Der Rückstand wird an Silikagel mit 30% Aethylacetat-Hexan chromatographiert. Man erhält 2,57 g (45%) 4-Brom-2-methyl-2-butanol, MS m/e 151 ($M^+$-$CH_3$).
b) Einer Lösung von 2,56 g 4-Brom-2-methyl-2-butanol und 4,86 g Imidazol in 15 ml N,N-dimethylformamid werden bei 0°C 6,48 g Chlortriäthylsilan zugesetzt. Das Gemisch wird 200 Minuten bei Raumtemperatur gerührt. Nach Zusatz von Eis wird das Gemisch mit Wasser verdünnt und mit Pentan extrahiert. Die organische Phase wird mit Wasser und gesättigter Salzlösung gewaschen, getrocknet und eingedampft. Der Rückstand wird an Silikagel mit Pentan chromatographiert. Man erhält 4,02 g (93%) (3-Brom-1,1-dimethylpropoxy)triäthylsilan, MS m/e 265 ($M^+$-$CH_3$).
c) Einer Lösung von 0,930 g [1(R*),3aR*(3aβ,4α,7aα]-3a,4,-5,6,7,7a-Hexahydro-4-hydroxy-β,7a-dimethyl-3H-inden-1-äthanol-4-methyl-benzosulfonat und 1,10 g Imidazol in 73 ml Methylenchlorid werden bei 0°C

0,580 g Chlortriäthylsilan zugesetzt. Das Gemisch wird 1,5 Stunden bei Raumtemperatur gerührt. Nach Zusatz von Eis wird das Gemisch mit Wasser verdünnt und 20 Minuten gerührt. Die organische Schicht wird mit Methylenchlorid extrahiert. Die Extrakte werden mit Wasser, 1N $H_2SO_4$, gesättigtes, wässriges $NaHCO_3$ und gesättigter Salzlösung gewaschen. Nach Trocknen und Eindampfen wird der Rückstand an Silikagel mit Aethylacetat-Hexan (1:5) gereinigt. Man erhält 1,22 g (100%) [1(R*),3aR*-(3aβ,4α,7aα)]-3a,4,5,6,7,7a-Hexahydro-4-[(triäthylsilyl)oxy]-β,7a-dimethyl-3H-inden-1-äthanol-4-methyl-benzosulfonat $[\alpha]_D^{20}$ + 46,1° (c 0,31, $CHCl_3$).

d) Einer Lösung von 3,08 g (3-Brom-1,1-dimethylpropoxy)-triäthylsilan in 31 ml THF werden 0,282 g Magnesium zugesetzt. Das Gemisch wird 3,5 Stunden bei 68°C erhitzt. Dann wird ein Gemisch von 0,686 g Kupfer(I)-Jodid zusammen mit der oben erwähnten Grignard-Lösung 30 Minuten bei 3°C gerührt. Eine Lösung von 1,02 g des Produkts von c) wird zugesetzt und das Gemisch wird 40 Minuten bei Raumtemperatur gerührt. Nach Zusatz eines Gemisches von Eis und Wasser wird das Gemisch mit Aether extrahiert. Die organische Phase wird mit 1N $H_2SO_4$ und gesättigter, wässriger $NaHCO_3$ gewaschen, getrocknet und eingedampft. Der Rückstand wird an Silikagel mit Aethylacetat-Hexan (1:15) chromatographiert. Man erhält 1,80 g [1(R*),3aR*-(3aβ,4α,7aα)]-3a,4,-5,6,7,7a-hexahydro-1-[1,5-dimethyl-5-[(triäthylsilyl)oxy]-hexyl]-4-[(triäthylsilyl)oxy]-7a-methyl-3H-inden, MS m/e 479 ($M^+$-Et).

e) Einer Lösung von 1,60 g des Produkts von d) in 5 ml THF werden 2,00 ml 1M Tetrabutylammoniumfluorid in THF zugesetzt. Das Gemisch wird 50 Minuten bei 68°C erhitzt. Nach Abkühlen auf Raumtempertur wird das Gemisch mit Wasser verdünnt und mit Methylenchlorid extrahiert. Die organische Phase wird mit einer Salzlösung gewaschen, getrocknet und eingedampft. Der Rückstand wird an Silikagel mit Aethylacetat-Hexan (1:1) gereinigt. Man erhält 0,420 g (79%) [1(R*),3aR*-(3aβ,4aα,7aα)]-3a,4,5,6,7,7a-hexahydro-4-hydroxy-a,α-ε,7a-tetramethyl-1H-inden-1-pentanol, $[\alpha]_D^{21}$ + 12,0° (c 0,25, $CHCl_3$).

f) Einer Lösung von 0,210 g des Produkts von e) in 18 ml Methylenchlorid werden 0,870 Pyridiniumdichromat und 44 mg Pyridinium-p-toluolsulfonat zugesetzt. Das Gemisch wird 175 Minuten gerührt. Nach Zusatz von 50 ml Aether wird das Gemisch 5 Minuten gerührt und filtriert. Die Feststoffe werden mit gesättigter wässriger $CuSO_4$-Lösung, Wasser, halb gesättigter, wässriger $NaHCO_3$ und gesättigter Salzlösung gewaschen. Die organische Phase wird getrocknet und eingedampft. Der Rückstand wird an Silikagel mit 35% Aethylacetat-Hexan chromatographiert. Man erhält 0,175 (84%) [1(R*),3aR*-(3aβ,7aα)]-3,3a,5,6,7,7a-Hexahydro-1-(5-hydroxy-1,5-dimethylhexyl)-7a-methyl-4H-inden-4-on, $[\alpha]_D^{21}$ +28,2° (c 0,22, $CHCl_3$).

g) Durch Behandlung von 0,168 g des Produkts von f) wie beschrieben in Beispiel 1j) erhält man 0,211 g (100%) [1(R*),3aR*-(3aβ,7aα)]-3,3a,5,6,7,7a-Hexahydro-1-(1,5-dimethyl-5-[(trimethylsilyl)oxy]hexyl)-7a-methyl-4H-inden-4-on, $[\alpha]_D^{20}$ + 21,9° (c 0,27, $CHCl_3$).

h) Wie in Beispiel 1k) beschrieben, jedoch ausgehend von 0,581 g [3S-(1Z,3α,5β)]-[2-[3,5-Bis[[(1,1-Dimethyläthyl)-dimethylsilyl]oxy]-2-methylencyclohexyliden]äthyl]diphenyl phosphinoxid und 0,210 g des Produkts von g) erhält man 0,358 g (83%) (1α,3β,5Z,7E)-1,3-Bis[[(1,1-dimethyläthyl)-dimethylsilyl]oxy]-25-[(trimethylsilyl)oxy]-9,10-secocholesta-5,7,10(19),16-tetraen, MS m/e 714 ($M^+$).

i) Behandlung von 0,350 g des Produkts von h) wie in Beispiel 1l) beschrieben ergibt 0,168 g (83%) 1,25-Dihydroxy-16-dehydrocholecalciferol, $[\alpha]_D^{20}$ +40,0°, (c 0,17, MeOH).

## Beispiel 6

a) Wie in Beispiel 1k) beschrieben erhält man ausgehend von 0,383 g [5S-(1Z)]-[2-[5-[[(1,1-Dimethyläthyl)dimethylsilyl]oxy]-2-methylencyclohexyliden]äthyl]diphenylphosphinoxid und 0,188 g des Produkts von Beispiel 5g) erhält man 0,245 g (78%) (3α,5Z,7E)-3-[[(1,1-Dimethyläthyl)-dimethylsilyl]oxy]-25-[(trimethylsilyl)oxy]-9,10-secocholesta-5,7,-10(19),16-tetraen, $[\alpha]_D^{24}$ +67,5° (c 0,20, $CHCl_3$).

b) Behandlung von 0,239 g des Produkts von a) wie in Beispiel 11) beschrieben ergibt 0,135 g (83%) 25-Hydroxy-16-dehydrocholecalciferol, $[\alpha]_D^{23}$ +75,4° (c = 0,13, MeOH).

In den folgenden Beispielen A und B ist die Zusammensetzung von Weichgelatinekapseln für die orale Verabreichung und von einer topisch verabreichbaren Creme angegeben:

Beispiel A

Beispiel A

| | mg/Kapsel |
|---|---|
| Verbindung E | 0.0001-0.010 |
| Butyliertes Hydroxytoluol | 0.016 |
| Butyliertes Hydroxyanisol | 0.016 |
| Fraktioniertes Kokosnussöl | 160.0 |

Beispiel B

Beispiel B

| | mg/g Creme |
|---|---|
| Verbindung E | 0.001-1.0 |
| Cetylalkohol | 1.5 |
| Stearylalkohol | 2.5 |
| Sorbitanmonostearat | 2.0 |
| Glycerylmonostearat und Polyoxyäthylenglyclolstearat | 4.0 |
| Polysorbat 60 | 1.0 |
| Mineralöl | 4.0 |
| Propylenglycol | 5.0 |
| Propylparaben | 0.05 |
| Butyliertes Hydroxyanisol | 0.05 |
| Sorbitollösung | 2.0 |
| EDTA-Dinatriumsalz | 0.01 |
| Methylparaben | 0.18 |
| Destilliertes Wasser | q.s. zu 100 g |

**Patentansprüche**

**Patentansprüche für folgende Vertragsstaaten: AT, BE, CH, DE, FR, GB, IT, LI, LU, NL, SE**

1. Eine Verbindung der Formel

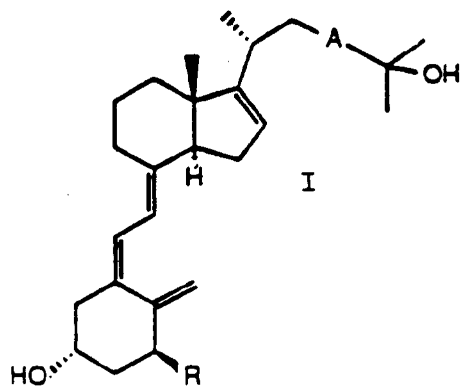

I

worin R Wasserstoff oder Hydroxy und A -C≡C-, -CH=CH- mit E-Konfiguration oder $-CH_2-CH_2-$ ist.

2. Eine Verbindung gemäss Anspruch 1 aus der Gruppe der folgenden:
1,25-Dihydroxy-16-dehydro-23-didehydrocholecalciferol,
1,25-Dihydroxy-16,23E-bisdehydrocholecalciferol und
1,25-Dihydroxy-16-dehydrocholecalciferol.

3. Eine Verbindung der Formel

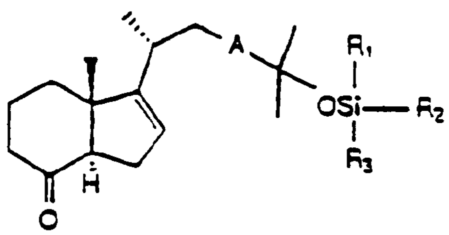

II

worin A -CH=CH- mit E-Konfiguration, $-CH_2-CH_2-$ oder insbesondere -C≡C-, und $R_1$ und $R_3$ unabhängig voneinander $C_{1-4}$-Alkyl, insbesondere Methyl, und $R_2$ $C_{1-4}$-Alkyl, insbesondere Methyl, Aryl oder Aryl-$C_{1-4}$-alkyl sind.

4. Eine Verbindung gemäss Anspruch 1 oder 2 zur Verwendung als therapeutisch aktive Substanz, insbesondere für die behandlung von hyperproliferativen Hautkrankheiten, insbesondere von Psoriasis, oder für die behandlung von neoplastischen Krankheiten, speziell von Leukämie.

5. Verfahren zur Herstellung einer Verbindung nach Anspruch 1 oder 2, dadurch gekennzeichnet, dass man eine entsprechende Verbindung der Formel I, die anstelle von zwei oder drei Hydroxygruppen, zwei bzw. drei geschützte Hydroxygruppen der Formel

$$-OSi(R_1,R_2,R_3)$$

enthält,

worin $R_1$ und $R_3$ $C_{1-4}$-Alkyl und $R_2$ $C_{1-4}$-Alkyl, Aryl oder Aryl-$C_{1-4}$-alkyl sind,

mit einem zur Entfernung der Schutzgruppen geeigneten Reagenz umsetzt.

6. Ein Medikament, insbesondere für die behandlung von hyperproliferativen Hautkrankheiten, speziell von Psoriasis, oder für die behandlung von neoplastischen Krankheiten, speziell Leukämie, bestehend aus einer wirksamen Menge einer Verbindung gemäss Anspruch 1 oder 2 und einem pharmazeutischem Trägermaterial, insbesondere für die orale oder topische Verabreichung.

7. Die Verwendung einer Verbindung gemäss Anspruch 1 oder 2 bei der Herstellung eines Medikaments für die behandlung von hyperproliferativen Hautkrankheiten, speziell von Psoriasis, oder für die behandlung von neoplastischen Krankheiten, speziell von Leukämie.

**Patentansprüche für folgenden Vertragsstaat: ES**

1. Verfahren zur Herstellung einer Verbindung der Formel

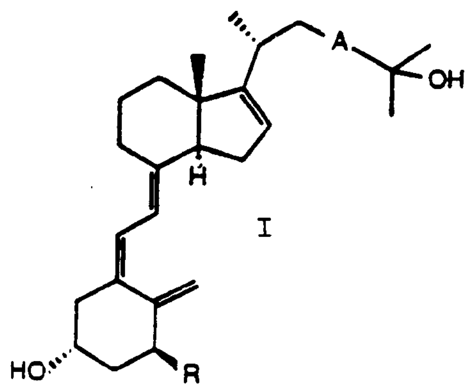

I

worin R Wasserstoff oder Hydroxy und A -C≡C-, -CH=CH- mit E-Konfiguration oder $CH_2$-$CH_2$- ist, dadurch gekennzeichnet, dass man eine entsprechende Verbindung der Formel I, die anstelle von zwei oder drei Hydroxygruppen, zwei bzw. drei geschützte Hydroxygruppen der Formel

$$-OSi(R_1,R_2,R_3)$$

enthält,

worin $R_1$ und $R_3$ $C_{1-4}$-Alkyl und $R_2$ $C_{1-4}$-Alkyl, Aryl oder Aryl-$C_{1-4}$-alkyl sind, mit einem zur Entfernung der Schutzgruppen geeigneten Reagenz umsetzt.

2. Verfahren nach Anspruch 1, dadurch gekennzeichnet, dass man eine Verbindung aus der Gruppe der folgenden herstellt;

1,25-Dihydroxy-16-dehydro-23-didehydrocholecalciferol,
1,25-Dihydroxy-16,23E-bisdehydrocholecalciferol und
1,25-Dihydroxy-16-dehydrocholecalciferol.

3. Verfahren nach Anspruch 1, dadurch gekennzeichnet, dass man die Schutzgruppen mit einem Fluorsalz in einem polaren, organischen Lösungsmittel abspaltet.

4. Verfahren zur Herstellung pharmazeutischer Präparate, enthaltend eine Verbindung der Formel I, dadurch gekennzeichnet, dass man eine solche Verbindung in eine pharmazeutische Anwendungsform bringt.

5. Eine Verbindung gemäss Anspruch 1 oder 2 zur Verwendung als therapeutisch aktive Substanz, insbesondere für die Behandlung von hyperproliferativen Hautkrankheiten, insbesondere von Psoriasis, oder für die Behandlung von neoplastischen Krankheiten, speziell von Leukämie.

**Patentansprüche für folgenden Vertragsstaat: GR**

1. Verfahren zur Herstellung einer Verbindung der Formel

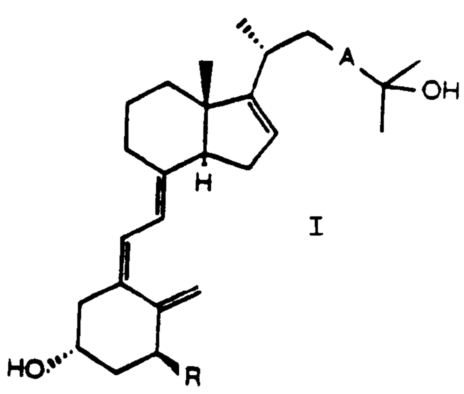

I

worin R Wasserstoff oder Hydroxy und A -C≡C-, -CH=CH- mit E-Konfiguration oder $CH_2$-$CH_2$- ist, dadurch gekennzeichnet ist, dass man eine entsprechende Verbindung der Formel I, die anstelle von zwei oder drei Hydroxygruppen, zwei bzw. drei geschützte Hydroxygruppen der formel

$$-OSi(R_1,R_2,R_3)$$

enthält,

worin $R_1$ und $R_3$ $C_{1-4}$-Alkyl und $R_2$ $C_{1-4}$-Alkyl, Aryl oder Aryl-$C_{1-4}$-alkyl sind,
mit einem zur Entfernung der Schutzgruppen geeigneten Reagenz umsetzt.

2. Verfahren nach Anspruch 1, dadurch gekennzeichnet, dass man eine Verbindung aus der Gruppe der folgenden herstellt:
1,25-Dihydroxy-16-dehydro-23-didehydrocholecalciferol,
1,25-Dihydroxy-16,23E-bisdehydrocholecalciferol und
1,25-Dihydroxy-16-dehydrocholecalciferol.

3. Verfahren zur Herstellung pharmazeutischer Präparate, enthaltend eine Verbindung der Formel I, dadurch gekennzeichnet, dass man eine solche Verbindung in eine pharmazeutische Anwendungsform bringt.

4. Eine Verbindung gemäss Anspruch 1 oder 2 zur Verwendung als therapeutisch aktive Substanz, insbesondere für die Behandlung von hyperproliferativen Hautkrankheiten, insbesondere von Psoriasis, oder für die Behandlung von neoplastischen Krankheiten, speziell von Leukämie.

5. Die Verwendung einer Verbindung gemäss Anspruch 1 oder 2 bei der Herstellung eines Medikaments für die Behandlung von hyperproliferativen Hautkrankheiten, speziell von Psoriasis, oder für die Behandlung von neoplastischen Krankheiten, speziell von Leukämie.

6. Eine Verbindung der Formel

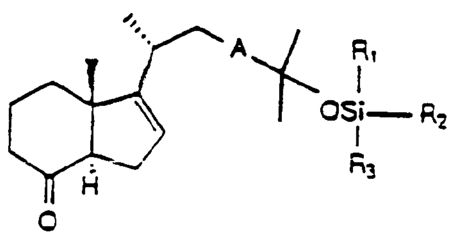

II

worin A -CH=CH- mit E-Konfiguration, $-CH_2-CH_2-$ oder insbesondere -C≡C-, und $R_1$ und $R_3$ unabhängig voneinander $C_{1-4}$-Alkyl, insbesondere Methyl, und $R_2$ $C_{1-4}$-Alkyl, insbesondere Methyl, Aryl oder Aryl-$C_{1-4}$-alkyl sind.

## Claims

### Claims for the following Contracting States: AT, BE, CH, DE, FR, GB, IT, LI, LU, NL, SE

1. A compound of the formula

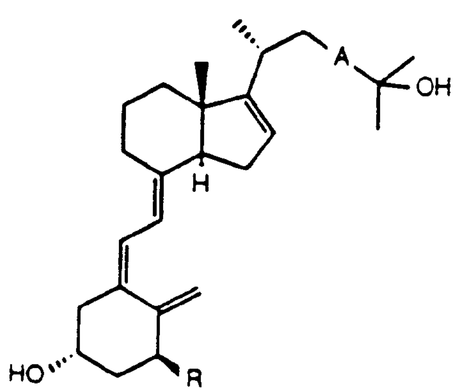

I

wherein R is hydrogen or hydroxy and A is -C≡C-, -CH=CH- with the E-configuration or $-CH_2-CH_2-$.

2. A compound in accordance with claim 1 from the following group:
1,25-Dihydroxy-16-dehydro-23-didehydrocholecalciferol,
1,25-dihydroxy-16,23E-bisdehydrocholecalciferol and
1,25-dihydroxy-16-dehydrocholecalciferol.

3. A compound of the formula

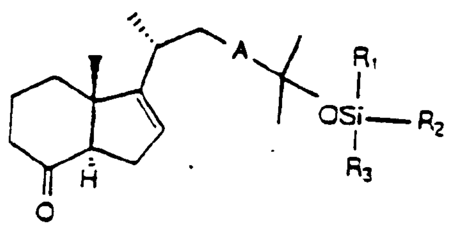

II

wherein A is -CH=CH- with the E-configuration, $-CH_2-CH_2-$ or especially -C≡C- and $R_1$ and $R_3$ are independently $C_{1-4}$-alkyl, especially methyl, and $R_2$ is $C_{1-4}$-alkyl, especially methyl, aryl or aryl-$C_{1-4}$-alkyl.

4. A compound in accordance with claim 1 or 2 for use as a therapeutically active substance, especially for the treatment of hyperproliferative skin diseases, particularly of psoriasis, or for the treatment of neoplastic diseases, particularly of leukaemia.

5. A process for the preparation of a compound according to claim 1 or 2, characterized by reacting a corresponding compound of formula I which contains instead of two or three hydroxy groups two or three protected hydroxy groups of the formula

$$-OSi(R_1,R_2,R_3)$$

wherein $R_1$ and $R_3$ are $C_{1-4}$-alkyl and $R_2$ is $C_{1-4}$-alkyl, aryl or aryl-$C_{1-4}$-alkyl,

with a reagent suitable for removing the protecting groups.

6. A medicament, especially for the treatment of hyperproliferative skin diseases, particularly of psoriasis, or for the treatment of neoplastic diseases, particularly leukaemia, comprising an effective amount of a compound according to claim 1 or 2 and a pharmaceutical carrier material, especially for oral or topical administration.

7. The use of a compound in accordance with claim 1 or 2 for the manufacture of a medicament for the treatment of hyperproliferative skin diseases, particularly of psoriasis, or for the treatment of neoplastic diseases, particularly of leukaemia.

**Claims for the following Contracting State: ES**

1. A process for the preparation of a compound of the formula

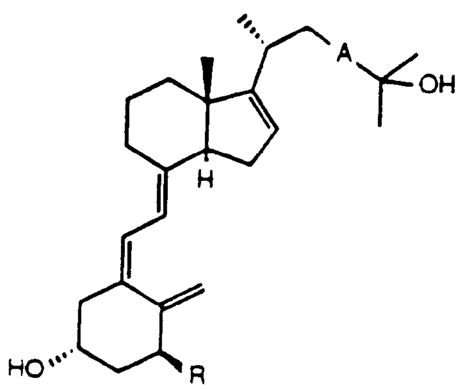

I

wherein R is hydrogen or hydroxy and A is -C≡C-, -CH=CH- with the E-configuration or $-CH_2-CH_2-$, characterized by reacting a corresponding compound of formula I which contains instead of two or three hydroxy groups two or three protected hydroxy groups of the formula

$$-OSi(R_1,R_2,R_3)$$

wherein $R_1$ and $R_3$ are $C_{1-4}$-alkyl and $R_2$ is $C_{1-4}$-alkyl, aryl or aryl-$C_{1-4}$-alkyl,

with a reagent suitable for removing the protecting groups.

2. A process according to claim 1, characterized in that a compound from the following group is prepared:
1,25-Dihydroxy-16-dehydro-23-didehydrocholecalciferol,
1,25-dihydroxy-16,23E-bisdehydrocholecalciferol and
1,25-dihydroxy-16-dehydrocholecalciferol

3. A process according to claim 1, characterized in that the protecting groups are cleaved off using a fluoride

salt in a polar, organic solvent.

4. A process for the preparation of pharmaceutical preparations containing a compound of formula I, characterized by bringing such a compound into a pharmaceutical administration form.

5. A compound in accordance with claim 1 or 2 for use as a therapeutically active substance, especially for the treatment of hyperproliferative skin diseases, particularly of psoriasis, or for the treatment of neoplastic diseases, particularly of leukaemia.

**Claims for the following Contracting State: GR**

1. A process for the preparation of a compound of the formula

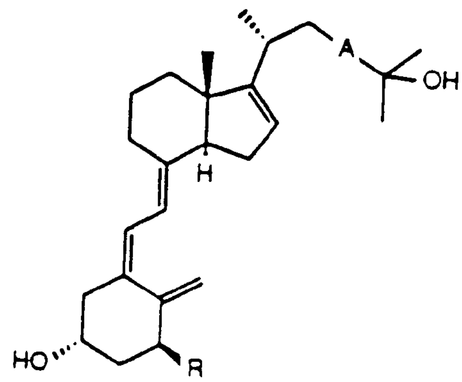

I

wherein R is hydrogen or hydroxy and A is -C≡C-, -CH=CH- with the E-configuration or $-CH_2-CH_2-$, characterized by reacting a corresponding compound of formula I which contains instead of two or three hydroxy groups two or three protected hydroxy groups of the formula

$$-OSi(R_1,R_2,R_3)$$

wherein $R_1$ and $R_3$ are $C_{1-4}$-alkyl and $R_2$ is $C_{1-4}$-alkyl, aryl or aryl-$C_{1-4}$-alkyl,
with a reagent suitable for removing the protecting groups.

2. A process according to claim 1, characterized in that a compound from the following group is prepared:
1,25- Dihydroxy-16-dehydro-23-didehydrocholecalciferol,
1,25-dihydroxy-16,23E-bisdehydrocholecalciferol and
1,25-dihydroxy-16-dehydrocholecalciferol

3. A process for the preparation of pharmaceutical preparations containing a compound of formula I, characterized by bringing such a compound into a pharmaceutical administration form.

4. A compound in accordance with claim 1 or 2 for use as a therapeutically active substance, especially for the treatment of hyperproliferative skin diseases, particularly of psoriasis, or for the treatment of neoplastic diseases, particularly of leukaemia.

5. The use of a compound in accordance with claim 1 or 2 for the manufacture of a medicament for the treatment of hyperproliferative skin diseases, particularly of psoriasis, or for the treatment of neoplastic diseases, particularly of leukaemia.

6. A compound of the formula

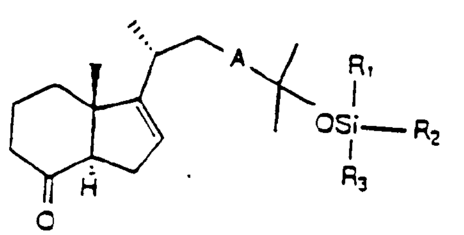

II

wherein A is -CH=CH- with the E-configuration, $-CH_2-CH_2-$ or especially -C≡C- and $R_1$ and $R_3$ are independently $C_{1-4}$-alkyl, especially methyl, and $R_2$ is $C_{1-4}$-alkyl, especially methyl, aryl or aryl-$C_{1-4}$-alkyl.

## Revendications

### Revendications pour les Etats contractants suivants: AT, BE, CH, DE, FR, GB, IT, LI, LU, NL, SE

1. Composé de formule

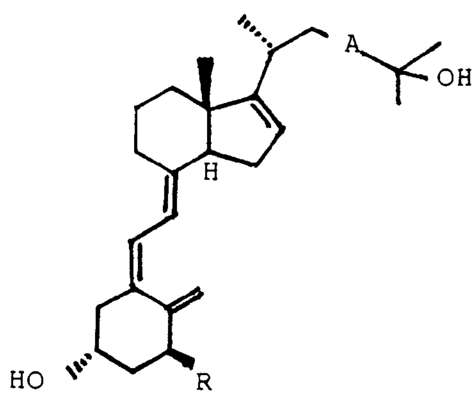

I

dans laquelle R est l'hydrogène ou un hydroxy et A est -C≡C-, -CH=CH- avec la configuration E ou -$CH_2$-$CH_2$-.

2. Composé selon la revendication 1 faisant partie du groupe suivant :
1,25-dihydroxy-16-déhydro-23-didéhydrocholécalciférol,
1,25-dihydroxy-16,23E-bisdéhydrocholécalciférol et
1,25-dihydroxy-16-déhydrocholécalciférol.

3. Composé de formule

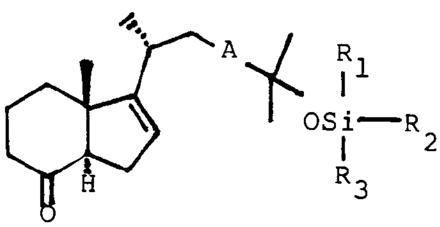

II

dans laquelle A est -CH=CH- avec la configuration E, -$CH_2$-$CH_2$- ou notamment -C≡C-, et $R_1$ et $R_3$ représentent indépendamment l'un de l'autre un alkyle $C_{1-4}$, notamment le méthyle, et $R_2$ un alkyle $C_{1-4}$, notamment le méthyle, un aryle ou aryl-alkyle $C_{1-4}$.

4. Composé selon la revendication 1 ou 2 pour l'utilisation en tant que substance thérapeutiquement active, notamment pour le traitement de maladies cutanées hyperproliférantes, notamment du psoriasis ou pour le traitement de maladies néoplastiques, spécialement de la leucémie.

5. Procédé de fabrication d'un composé selon la revendication 1 ou 2, caractérisé en ce qu'on fait réagir un composé correspondant à la formule I, qui comprenne à la place de deux ou trois groupes hydroxy, deux ou trois groupes hydroxy protégés de formule

$$-OSi(R_1,R_2,R_3)$$

dans laquelle $R_1$ et $R_3$ sont des alkyles $C_{1-4}$ et $R_2$ des alkyle $C_{1-4}$, aryle ou arylalkyle $C_{1-4}$, avec un réactif approprié à l'élimination des groupes protecteurs.

6. Médicament, notamment pour le traitement de maladies cutanées hyperproliférantes, spécialement de psoriasis, ou pour le traitement de maladies néoplastiques, spécialement de la leucémie, comprenant une quantité efficace d'un composé selon les revendications 1 ou 2 et d'un matériau véhicule pharmaceutique, notamment pour l'administration orale ou topique.

7. Utilisation d'un composé selon la revendication 1 ou 2 pour préparer un médicament pour le traitement des maladies cutanées hyperproliférantes, spécialement du psoriasis, où pour le traitement de maladies néoplastiques, spécialement de la leucémie.

**Revendications pour l'Etat contractant suivant: ES**

1. Procédé de préparation d'un composé de formule :

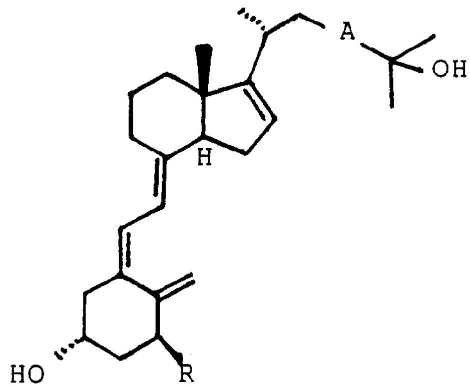

I

dans laquelle R est l'hydrogène ou un hydroxy et A est -C≡C-, -CH=CH- avec la configuration E ou est $-CH_2-CH_2-$, caractérisé en ce qu'on fait réagir un composé correspondant à la formule I, qui comprenne à la place de deux ou trois groupes hydroxy, deux ou trois groupes hydroxy protégés de formule

$-OSi(R_1,R_2,R_3)$

dans laquelle $R_1$ et $R_3$ sont des alkyles $C_{1-4}$ et $R_2$ des alkyle $C_{1-4}$, aryle ou aryl-alkyle $C_{1-4}$,
avec un réactif approprié à l'élimination des groupes protecteurs.

2. Procédé selon la revendication 1, caractérisé en ce qu'on prépare un composé faisant partie du groupe suivant :

1,25-dihydroxy-16-déhydro-23-didéhydrocholécalciférol,
1,25-dihydroxy-16,23E-bisdyhydrocholécalciférol et
1,25-dihydroxy-16-déhydrocholycalciférol.

3. Procédé selon la revendication 1, caractérisé en ce qu'on élimine les groupes protecteurs avec un sel fluoré dans un solvant organique polaire.

4. Procédé de fabrication de préparations pharmaceutiques, comprenant un composé de formule I, caractérisé en ce qu'on place un tel composé sous une forme d'administration pharmaceutique.

5. Composé selon la revendication 1 ou 2 pour l'utilisation en tant que substance thérapeutiquement active, notamment pour le traitement de maladies cutanées hyperproliférantes, notamment du psoriasis ou pour le traitement de maladies néoplastiques, spécialement de la leucémie.

**Revendications pour l'Etat contractant suivant: GR**

1. Procédé de préparation d'un composé de formule

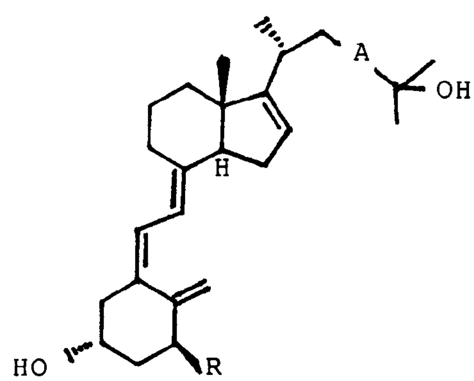

I

dans laquelle R est l'hydrogène ou un hydroxy et A est -C≡C-, -CH=CH- avec la configuration E ou est $-CH_2-CH_2-$, caractérisé en ce qu'on fait réagir un composé correspondant à la formule I, qui comprenne à la place de deux ou trois groupes hydroxy, deux ou trois groupes hydroxy protégés de formule

$-OSi(R_1,R_2,R_3)$

dans laquelle $R_1$ et $R_3$ sont des alkyles $C_{1-4}$ et $R_2$ des alkyle $C_{1-4}$, aryl ou aryl-alkyle $C_{1-4}$, avec un réactif approprié à l'élimination des groupes protecteurs.

2. Procédé selon la revendication 1, caractérisé en ce qu'on prépare un composé faisant partie du groupe suivant :

1,25-dihydroxy-16-dehydro-23-didéhydrocholécalciférol,
1,25-dihydroxy-16,23E-bisdehydrocholécalciferol et
1,25-dihydroxy-16-déhydrocholécalciférol.

3. Procédé de fabrication de préparations pharmaceutiques, comprenant un composé de formule I, caractérisé en ce qu'on met un tel composé sous une forme d'administration pharmaceutique.

4. Composé selon la revendication 1 ou 2 pour l'utilisation en tant que substance thérapeutiquement active, notamment pour le traitement de maladies cutanées hyperproliférantes, notamment du psoriasis ou pour le traitement de maladies néoplastiques, spécialement de la leucémie.

5. Utilisation d'un composé selon la revendication 1 ou 2 pour produire un médicament pour le traitement de maladies cutanées hyperproliférantes, spécialement le psoriasis ou pour le traitement de maladies néoplastiques, spécialement de la leucémie.

6. Composé de formule

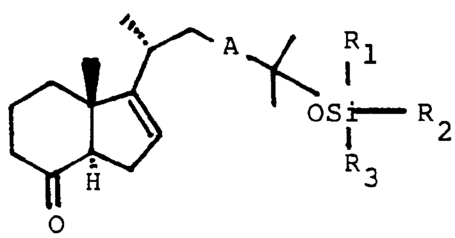

II

dans laquelle A est -CH=CH- avec la configuration E, $-CH_2-CH_2-$ ou notamment -C≡C-, et $R_1$ et $R_3$ représentent indépendamment l'un de l'autre un alkyle $C_{1-4}$, notamment le méthyle, et $R_2$ un alkyle $C_{1-4}$, notamment le méthyle, un aryle ou aryl-alkyle $C_{1-4}$.